# EUROPEAN PATENT APPLICATION

(11) **EP 3 838 283 A1**
(43) Date of publication of application: **23.06.2021**
(21) Application number: 19217433.2
(22) Date of filing: 18.12.2019
(51) Int. Cl.: A61K 36/324, A61K 31/19, A61K 31/4188, A61K 31/592, A61K 31/593, A61K 31/714, A61K 33/04, A61K 33/34, A61P 37/00, A61P 29/00

(54) **COMPOSITION FOR USE IN THE TREATMENT OF PROVOCATIVE DISEASES**

(71) Applicant: Mundus Sanus GmbH & Co. KG, 83527 Kirchdorf (DE)
(72) Inventor: RUPPENTHAL, Christian, 83527 Kirchdorf (DE); EGGER, Carsten, 83527 Kirchdorf (DE); WOLFF, Detlef, 83527 Kirchdorf (DE)
(74) Representative: Vos, Derk

(57) **Abstract**

The present invention relates to a composition for use in the treatment or amelioration of an inflammatory or autoimmune disease, wherein the composition comprises Boswellia, a propionate, vitamin D, biotin, and vitamin B₁₂.

## Description

### BACKGROUND OF THE INVENTION

### Field of the invention

The present invention relates to a composition for use in the treatment or amelioration of inflammatory or autoimmune diseases.

### Background

Chronic inflammatory or autoimmune diseases may have different course. The symptoms associated therewith can only partly be reduced or ceased with the currently available immune-modulating or immune-suppressing drug based therapies.

Tiredness and fatigue are often the result of these inflammatory reactions, in particular in multiple sclerosis, but are also known as side effects of the drugs used (e.g., for interferon therapies). Existing therapies rarely guarantee "Freedom of Disease Status", and there is a high need for "More" (without additional side effects). Patients diagnosed with a chronic inflammatory disease, such as multiple sclerosis, still having minor symptoms are sometimes unwilling to take the existing drugs due to the partly serious side effects.

### SUMMARY

There is thus a need for the treatment or amelioration of a chronic inflammatory or autoimmune disease, such as multiple sclerosis, having less or no side effects, which therapy is well tolerated and accepted by the patients, and which increases the general condition of a patient.

It was found that patients having a chronic inflammatory or autoimmune disease frequently additionally have an increased need for vitamins and trace elements in order to guarantee the normal metabolism. Studies show that in patients having a chronic autoimmune disease, the intestinal activity often does not correspond to that of a healthy person. Since normal intestinal function plays an important role for the immune system, the promotion of normal function is a possible starting point for the treatment of an anti-inflammatory or autoimmune disease. Existing therapies have no or insufficient effect here.

The present invention relates to a composition for use in the treatment or amelioration of an inflammatory or autoimmune disease, wherein the composition comprises Boswellia, a propionate, vitamin D, biotin, and vitamin B₁₂. It was surprisingly found that a composition containing at least these ingredients is useful in the treatment or amelioration of inflammatory or autoimmune disease, in particular multiple sclerosis.

The details and further aspects are disclosed below.

### DETAILED DESCRIPTION

In a first aspect, the present disclosure relates to a composition for use in the treatment or amelioration of an inflammatory or autoimmune disease, wherein the composition comprises:
i) Boswellia;
ii) Propionate;
iii) Vitamin D;
iv) Biotin; and
v) Vitamin B₁₂.

The inventors of the present disclosure surprisingly found that a combination of the compounds Boswellia, propionate, vitamin D, biotin, and vitamin B₁₂ may be used to treat or ameliorate certain diseases or conditions. In particular, the composition of the present disclosure may be used to treat or ameliorate inflammatory or autoimmune diseases, such as multiple sclerosis. The composition of the present disclosure is a pharmaceutical composition, and it may thus also be referred to as pharmaceutical composition.

The composition may be used to treat a human or animal body by administering the composition to any human or animal that may experience the beneficial effects of the composition. Foremost such animals are mammals, e.g., humans and companion animals, although it is not intended to be limited in this respect.

Methods of administration may include oral administration. In a preferred embodiment, delivery is systemic.

While it is thought that boswellic acids are the bioactive principle of frankincense, which is reported to reduce disease activity in relapsing-remitting multiple sclerosis (MS) (Stürner KH, et al. J Neurol Neurosurg Psychiatry 2018;89:330-338), it is assumed that the compounds or ingredients of the present composition in their specific combination have a synergistic effect over the single use of frankincense as reported by Stürner. The synergistic effect may be expressed in reduced disease activity in patients, especially with relapsing-remitting multiple sclerosis (RRMs). Disease activity measures can be the number and volume of contrast-enhancing lesions (ceL) measured in MRI. It may also be possible that the progression of the disease is not accelerated, slowed down, or even stopped.

Without being bound by any theory, it is assumed that the synergistic effect of the composition of the present disclosure is also expressed in reduced amounts of the individual compounds of the composition, compared to a treatment with only said single individual compound. While in some cases, individual compounds are applied at high doses, these doses can be reduced in the composition of the present disclosure, but still achieving an effect similar or comparable to the effect achieved by the individual compound in higher (maximal) dose. Thereby, the frequency and/or severity of possible side effects can be reduced.

In another embodiment of the first aspect, the inflammatory or autoimmune disease is a chronic inflammatory disease, and/or the disease is selected from the group consisting of multiple sclerosis, colitis ulcerosa, Crohn's disease, cystic fibrosis, rheumatoid artritis, osteoarthritis, autoimmune diseases, inflammatory bowel disease, acute respiratory distress syndrome (ARDS) and asthma bronchiale. In a particularly preferred embodiment, the disease is multiple sclerosis.

In another embodiment of the first aspect, the composition for use comprises Boswellia, preferably a dry extract of *Boswellia Serrata,* in an amount of from 30 to 1300 mg, preferably from 60 to 1000 mg, further preferably from 130 to 600 mg, still further preferably from 200 to 500 mg, still further preferably from 300 to 450 mg, and still further preferably from 350 to 430 mg, and most preferably about 400 mg. In the context of the present disclosure, Boswellia refers to any kind of Boswellia, including a resin, gum resin or dry extract. In a particularly preferred embodiment, the Boswellia contains boswellic acid(s) in an amount of 60 to 95 % by weight, preferably 65 to 90 % by weight, and still further preferably 70 to 85 % by weight.

In another embodiment of the first aspect, the propionate in the composition for use is selected from the group consisting of sodium propionate, potassium propionate, and calcium di-propionate, and preferably wherein the propionate is sodium propionate. In general, the type of propionate is not relevant for the composition, as long as the propionate is in a form which can be readily absorbed by the human or animal body.

Propionic acid, and therewith also propionate, is considered as a short chain fatty acid, and it may have a positive effect on the immune system, in particular if the immune system is in dysbalance. Furthermore, propionic acid may have a positive effect on the increase of anti-inflammatory regulatory T cells, and it may reduce the number of inflammatory agents, such as IL17 and/or TNT alpha, by up to 50 %, and the number of sentinel T lymphocytes may be increased by up to 30 %.

In another embodiment of the first aspect, the composition comprises a propionate, preferably sodium propionate, in an amount of from 30 to 600 mg, preferably from 100 to 300 mg, further preferably from 130 to 250 mg, still further preferably from 150 to 230 mg, still further preferably from 150 to 200 mg, and most preferably about 180 mg, given as an amount of sodium propionate.

In another embodiment of the first aspect, the composition comprises vitamin D in an amount of from 1 to 15 µg, preferably from 1.5 to 13 µg, further preferably from 3 to 11 µg, still further preferably from 6 to 10 µg, and most preferably about 8.3 µg. Vitamin D may add to the normal function of the immune system, and has an influence on the cell division.

In another embodiment of the first aspect, the composition comprises biotin in an amount of from 3 to 60 µg, preferably from 15 to 50 µg, further preferably from 25 to 40 µm, and most preferably about 30 µg. Biotin may add to a normal function of the immune system and the metabolism.

In another embodiment of the first aspect, the composition comprises vitamin B₁₂ in an amount of from 0.2 to 3 µg, preferably from 0.4 to 2 µg, still further preferably from 0.6 to 1 µg, and most preferably about 0.83 µg.

Apart from the essential compounds, the composition of the present disclosure may contain other ingredients, such as zinc, vitamin B₂, vitamin B₆, copper and selenium. Accordingly, in another embodiment of the first aspect, the composition comprises further comprises at least one compound selected from the group consisting of zinc, vitamin B₂, vitamin B₆, copper and selenium. Vitamin B₂ may protect cells from oxidative stress, vitamin B₆ may add to a normal function of the immune system, and vitamin B₁₂ may have a regulatory function during cell division. All together these B-vitamins may add to a normal function of the nerve system, to a normal energy metabolism, and they may reduce fatigue and tiredness.

In another embodiment of the first aspect, the composition comprises zinc which is present as zinc oxide (ZnO), zinc acetate (ZnOAc), zinc citrate, zinc gluconate and/or zinc sulfate (ZnSO₄). In general, the zinc may be added to the composition in any form, as long as the zinc is in a form which can be readily absorbed by the human or animal body. Preferably, zinc may be added as salt of zinc(II). Zinc may have an influence on the function of cell division, and it may add to a normal fatty acid metabolism, as well as to a normal cognitive function.

In another embodiment of the first aspect, the composition comprises copper which is present as copper(II) sulfate (CuSO₄), copper(II) gluconate, copper(II) citrate, and/or copper(II) carbonate hydroxide (Cu₂CO₃(OH)₂). In general, the copper may be added to the composition in any form, as long as the copper is in a form which can be readily absorbed by the human or animal body. Preferably, copper may be added as salt of copper(II). Copper may also add to a normal function of the immune system, and it may protect cells from oxidative stress.

In another embodiment of the first aspect, the composition comprises selenium which is present as selenium-enriched yeast and/or sodium selenite (Na₂SeO₃). In general, the selenium may be added to the composition in any form, as long as the selenium is in a form which can be readily absorbed by the human or animal body. Selenium may add to a normal function of the immune system, and it may protect cells from oxidative stress.

In another embodiment of the first aspect, the composition comprises vitamin B₂ and/or vitamin B₆, each individually, in an amount of from 0.1 to 2 mg, preferably from 0.2 to 1.5 mg, still further preferably from 0.3 to 1 mg, and most preferably about 0.46 mg.

In another embodiment of the first aspect, the composition comprises zinc in an amount of from 1 to 10 mg, preferably from 2 to 5 mg, still further preferably from 3 to 4 mg, and most preferably about 3.5 mg. The amount of zinc is given as "pure" zinc, referring to the amount of zinc in atomic state, and not to the total amount of the salt. Accordingly, if, e.g., an amount of 65.38 mg zinc are used, and the zinc is added as zinc oxide, then 81.39 mg of zinc oxide are added.

In another embodiment of the first aspect, the composition comprises copper in an amount of from 100 to 1000 µg, preferably from 200 to 500 µg, still further preferably from 300 to 350 µg, and most preferably about 333 µg. The amount of copper is given as "pure" copper, referring to the amount of copper in atomic state, and not to the total amount of the salt. Accordingly, if, e.g., an amount of 63.55 mg copper are used, and copper zinc is added as copper sulfate, then 159.61 mg of (water free) copper sulfate are added.

In another embodiment of the first aspect, the composition further comprises an excipient selected from the group consisting of hydroxypropyl methyl cellulose and rice extract. The rice extract acts as a physical release agent in the composition of the present disclosure.

Pharmaceutical compositions of the present disclosure can take the form of tablets, pills, pellets, powders, multi-particulates, capsules, capsules containing powders, and capsules containing multi-particulates. In one embodiment, the composition is in the form of a tablet. In another embodiment, the composition is in the form of a capsule. Other examples of suitable pharmaceutical excipients are described in Remington's Pharmaceutical Sciences 1447-1676 (Alfonso R. Gennaro ed., 19th ed. 1995). Pharmaceutical compositions of the invention preferably comprise a suitable amount of a pharmaceutically acceptable excipient so as to provide the form for proper administration to the patient. Such a pharmaceutical excipient can be a diluent, suspending agent, solubilizer, binder, disintegrant, preservative, coloring agent, lubricant, and the like. The pharmaceutical excipient can be a liquid, such as water or an oil, including those of petroleum, animal, vegetable, or synthetic origin, such as peanut oil, soybean oil, mineral oil, sesame oil, and the like. The pharmaceutical excipient can be saline, gum acacia, gelatin, starch paste, talc, keratin, colloidal silica, urea, and the like. In addition, auxiliary, stabilizing, thickening, lubricating, and coloring agents can be used. In one embodiment, the pharmaceutically acceptable excipient is sterile when administered to a patient. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like.

The compositions, if desired, can also contain minor amounts of wetting or emulsifying agents, or pH buffering agents. Specific examples of pharmaceutically acceptable carriers and excipients that can be used to formulate oral dosage forms are described in the Handbook of Pharmaceutical Excipients, American Pharmaceutical Association (1986). In certain embodiments, the composition is formulated for oral administration. A composition to be orally delivered can be in the form of tablets, capsules, gelcaps, caplets, lozenges, aqueous or oily solutions, suspensions, granules, powders, emulsions, syrups, or elixirs, for example. An orally administered composition can contain one or more additional agents such as, for example, sweetening agents such as fructose, aspartame or saccharin; flavoring agents such as peppermint, oil of wintergreen, or cherry; coloring agents; and preserving agents, and stabilizers, to provide stable, pharmaceutically palatable dosage forms. Techniques and compositions for making solid oral dosage forms are described in Pharmaceutical Dosage Forms: Tablets (Lieberman, Lachman and Schwartz, eds., 2nd ed.) published by Marcel Dekker, Inc. Techniques and compositions for making tablets (compressed and molded), capsules (hard and soft gelatin) and pills are also described in Remington's Pharmaceutical Sciences 1553-1593 (Arthur Osol, ed., 16th ed., Mack Publishing, Easton, PA 1980).

In another embodiment of the first aspect, the composition for use is present as a powder. Said powder may advantageously be encapsulated in a capsule, preferably made of hydroxypropyl methyl cellulose.

In another embodiment of the first aspect, the composition for use is a dosage form in the form of a capsule or tablet, preferably a capsule, further preferably made of hydroxypropyl methyl cellulose.

In another embodiment of the first aspect, the daily dose for a human comprises one to three, preferably three dosage forms.

### Definitions

Where the term "comprising" is used in the present description and claims, it does not exclude other elements. For the purposes of the present invention, the term "consisting of' is considered to be a preferred embodiment of the term "comprising". If hereinafter a group is defined to comprise at least a certain number of elements, this is also to be understood to disclose a group, which preferably consists only of these elements.

Where an indefinite or definite article is used when referring to a singular noun, e.g. "a", "an" or "the", this includes a plural of that noun unless specifically stated otherwise.

Terms like "obtainable" or "definable" and "obtained" or "defined" are used interchangeably. This, e.g., means that, unless the context clearly dictates otherwise, the term "obtained" does not mean to indicate that e.g. an embodiment must be obtained by e.g. the sequence of steps following the term "obtained" even though such a limited understanding is always included by the terms "obtained" or "defined" as a preferred embodiment.

The term "Boswellia" refers to an extract or gum resin, together referred to as "resin" herein, obtained from a tree of the genus *Boswellia.* Preferred embodiments of the genus *Boswellia* are *Boswellia Serrata, Boswellia Frereana, Boswellia Papyrifera,* and *Boswellia Sacra.* Particularly preferred are *Boswellia Serrata* and *Boswellia Sacra,* with *Boswellia Serrata* being the most preferred.

The resin obtained from *Boswellia* contains boswellic acids, a series of pentacyclic triterpene molecules that are produced by plants in the genus *Boswellia.* Boswellic acids appear in the resin of the plant that exudes them; it is estimated that they make up 30 % of the resin of *Boswellia serrata.* While boswellic acids are a major component of the resin, the steam or hydro distilled frankincense essential oil does not contain any boswellic acid as these components are non-volatile and too large to come over in the steam distillation process (the essential oil is composed mainly of the much lighter monoterpene and sesquiterpene molecules with small amounts of diterpenoid components being the upper limit in terms of molecular weight). The amount of boswellic acids is considered to be particularly high in resins obtained from *Boswellia Serrata.* Since it is assumed that boswellic acids are mainly responsible for the therapeutic effect associated with Boswellia, it is preferred to use a resin having a high amount of boswellic acids.

The resin of *Boswellia* species has been used as incense in religious and cultural ceremonies and in medicines since time immemorial. *Boswellia Serrata* (Salai/Salai guggul), is a moderate to large sized branching tree of family *Burseraceae* (Genus *Boswellia*), grows in dry mountainous regions of India, Northern Africa and Middle East. Oleo gum-resin is tapped from the incision made on the trunk of the tree and is then stored in specially made bamboo basket for removal of oil content and getting the resin solidified. After processing, the gum-resin is then graded according to its flavour, colour, shape and size. In India, the States of Andhra Pradesh, Gujarat, Madhya Pradesh, Jharkhand and Chhattisgarh are the main source of *Boswellia Serrata.* Regionally, it is also known by different names. The oleo gum-resins contain 30-60% resin, 5-10% essential oils, which are soluble in the organic solvents, and the rest is made up of polysaccharides. Gum-resin extracts of *Boswellia Serrata* have been traditionally used in folk medicine for centuries to treat various chronic inflammatory diseases. The resinous part of *Boswellia Serrata* possesses monoterpenes, diterpenes, triterpenes, tetracyclic triterpenic acids and four major pentacyclic triterpenic acids, i.e. β-boswellic acid, acetyl-β-boswellic acid, 11-keto-β-boswellic acid and acetyl-11-keto-β-boswellic acid, responsible for inhibition of pro-inflammatory enzymes. Out of these four boswellic acids, acetyl-11-keto-β-boswellic acid is the most potent inhibitor of 5-lipoxygenase, an enzyme responsible for inflammation.

The resin of *Boswellia* may as well contain α-boswellic acid, and derivatives thereof, such as acetyl-11-keto-α-boswellic acid.

The purity of *Boswellia* resin refers to the relative amount of boswellic acids. The amount of boswellic acids is given in wt.-%, with the remainder being other compounds, such as, e.g., essential oils, and proteins, which are usually not referred to as impurities. Currently available resins of *Boswellia* have a purity in the range of about 60 to 85 % by weight.

In the context of the present disclosure, it is preferred to use a resin of *Boswellia* having a high purity, in particular a purity of 60 % by weight and more, or 70 % by weight and more, or 80 % by weight and more, or 85 % by weight and more. The purity of Boswellia may be determined in accordance with Ph. Eur., 8. Ausgabe, Grundwerk 2014, 2310 "Indischer Weihrauch".

In a particularly preferred embodiment, Boswellia is used as an extract of a resin or gum resin, in particular in the form of a dry extract. The resin or gum resin may be extracted using lipophilic solvents, such as solvents of class 2 and/or 3 according to Ph. Eur., addition 1999, chapter 5.4. It is particularly preferred to use a dry extract of *Boswellia Serrata* in the context of the present disclosure, i.e., in the composition for use.

The "propionate", also termed "propanoate", as used herein is the salt or ester of propionic acid, CH₃CH₂COOH. The propionate is preferably in the form of a salt, in particular the sodium, potassium or calcium salt. The sodium salt, i.e., sodium propionate, is particularly preferred.

The "vitamin D" as used herein may comprise vitamins D₁, D₂, D₃, D₄, and D₅, and in particular comprises vitamins D₂ and D₃, and most preferably vitamin D₃, also referred to as Cholecalciferol. For use in the treatment or amelioration of humans, an amount of 1 µg vitamin D corresponds to 40 International Units (IU).

The "biotin" as used herein is also referred to as vitamin H or vitamin B₇.

The "vitamin B₁₂" as used herein is also referred to as cobalamin. In a preferred embodiment, vitamin B₁₂ is used in the form of methylcobalamin.

### EXAMPLE

### Example

The corresponding compounds were provided as powder, mixed in appropriate amounts, and filled into a HPMC capsule, such that a capsule contains the following amounts:

| **Content of substances** | 1 capsule |
|---|---|
| Boswellia serrata 85% | 400 mg |
| Natrium propionate | 180 mg |
| Vitamin D3 | 333,33 IU |
| Vitamin B7 | 30 µg |
| Zinc | 3,5 mg |
| Copper | 333 µg |
| Selenium | 18 µg |
| Vitamin B2 | 0,47 mg |
| Vitamin B6 | 0,47 mg |
| Vitamin B12 | 0,83 µg |

## Claims

1. Composition for use in the treatment or amelioration of an inflammatory or autoimmune disease, wherein the composition comprises:
i) Boswellia;
ii) Propionate;
iii) Vitamin D;
iv) Biotin; and
v) Vitamin B₁₂.

2. Composition for use according to claim 1, wherein the inflammatory or autoimmune disease is a chronic inflammatory disease, and/or wherein the disease is selected from the group consisting of multiple sclerosis, colitis ulcerosa, Crohn's disease, cystic fibrosis, rheumatoid artritis, osteoarthritis, autoimmune diseases, inflammatory bowel disease, acute respiratory distress syndrome (ARDS) and asthma bronchiale.

3. The composition for use according to any one of claims 1 to 2, wherein the composition comprises Boswellia, preferably a dry extract of *Boswellia Serrata,* in an amount of from 30 to 1300 mg, preferably from 60 to 1000 mg, further preferably from 130 to 600 mg, still further preferably from 200 to 500 mg, still further preferably from 300 to 450 mg, and still further preferably from 350 to 430 mg, and most preferably about 400 mg.

4. The composition for use according to any one of claims 1 to 3, wherein the propionate is selected from the group consisting of sodium propionate, potassium propionate, and calcium di-propionate, and preferably wherein the propionate is sodium propionate.

5. The composition for use according to any one of claims 1 to 4, wherein the composition comprises a propionate, preferably sodium propionate, in an amount of from 30 to 600 mg, preferably from 100 to 300 mg, further preferably from 130 to 250 mg, still further preferably from 150 to 230 mg, still further preferably from 150 to 200 mg, and most preferably about 180 mg.

6. The composition for use according to any one of claims 1 to 5, wherein the composition comprises vitamin D in an amount of from 1 to 15 µg, preferably from 1.5 to 13 µg, further preferably from 3 to 11 µg, still further preferably from 6 to 10 µg, and most preferably about 8.3 µg.

7. The composition for use according to any one of claims 1 to 6, wherein the composition comprises biotin in an amount of from 3 to 60 µg, preferably from 15 to 50 µg, further preferably from 25 to 40 µm, and most preferably about 30 µg; and/or wherein the composition comprises vitamin B₁₂ in an amount of from 0.2 to 3 µg, preferably from 0.4 to 2 µg, still further preferably from 0.6 to 1 µg, and most preferably about 0.83 µg.

8. The composition for use according to any one of claims 1 to 7, wherein the composition further comprises at least one compound selected from the group consisting of zinc, vitamin B₂, vitamin B₆, copper and selenium.

9. The composition for use according to claim 8, wherein the zinc is present as zinc oxide (ZnO), zinc acetate (ZnOAc), zinc citrate, zinc gluconate and/or zinc sulfate (ZnSO₄); and/or wherein the copper is present as copper(II) sulfate (CuSO₄), copper(II) gluconate, copper(II) citrate, and/or copper(II) carbonate hydroxide (Cu₂CO₃(OH)₂); and/or wherein the selenium is present as selenium-enriched yeast and/or sodium selenite (Na₂SeO₃).

10. The composition for use according to any one of claims 8 to 9, wherein the composition comprises vitamin B₂ and/or vitamin B₆, each individually, in an amount of from 0.1 to 2 mg, preferably from 0.2 to 1.5 mg, still further preferably from 0.3 to 1 mg, and most preferably about 0.46 mg.

11. The composition for use according to any one of claims 8 to 10, wherein the composition comprises zinc in an amount of from 1 to 10 mg, preferably from 2 to 5 mg, still further preferably from 3 to 4 mg, and most preferably about 3.5 mg; and/or wherein the composition comprises copper in an amount of from 100 to 1000 µg, preferably from 200 to 500 µg, still further preferably from 300 to 350 µg, and most preferably about 333 µg.

12. The composition for use according to any one of claims 1 to 11, wherein the composition further comprises an excipient selected from the group consisting of hydroxypropyl methyl cellulose and rice extract.

13. The composition for use according to any one of claims 1 to 12, wherein the composition is a dosage form in the form of a capsule or tablet.

14. The composition for use according to claim 13, wherein the daily dose for a human comprises one to three, preferably three dosage forms.
